# EUROPEAN PATENT SPECIFICATION

(11) **EP 1 181 934 B1**
(45) Date of publication and mention of the grant of the patent: **06.10.2004**
(21) Application number: 00915483.2
(22) Date of filing: 11.04.2000
(51) Int. Cl.: A61K 31/496, A61P 27/02

(54) **1,4-DIHYDROPYRIDINE DERIVATIVES AS INHIBITORS FOR OPTIC HYPOFUNCTION CAUSED BY OPTIC NERVE CELL INJURY INDUCED BY FACTORS OTHER THAN OPTIC CIRCULATORY DISORDER**
1,4-dihydropyridinderivate als Inhibitoren einer Sehunterfunktion, hervorgerufen durch eine Sehnervzellenverletzung, die durch andere Faktoren als optischen Durchblutungstörungen induziert wird.
DERIVES DE1,4-DIHYDROPYRIDINE COMME INHIBITEURS D'HYPOFONCTION OPTIQUE PROVOQUEE PAR UNE LESION D'UNE CELLULE DU NERF OPTIQUE INDUITE PAR DES FACTEURS AUTRES QUE LES TROUBLES CIRCULATOIRES OPTIQUES

(30) Priority: 14.04.1999 JP 10642599
(43) Date of publication of application: 27.02.2002
(73) Proprietor: Senju Pharmaceutical Co., Ltd., Osaka-shi, Osaka 541-0046 (JP); Kyoto Pharmaceutical Industries, Ltd., Kyoto-shi, Kyoto 604-8444 (JP)
(72) Inventor: AZUMA, Mitsuyoshi, Nishinomiya-shi, Hyogo 662-0031 (JP); YOSHIDA, Yukuo, Kobe-shi, Hyogo 651-1203 (JP); SAKAMOTO, Yuji, Kobe-shi, Hyogo 651-2116 (JP)
(74) Representative: von Kreisler, Alek, Dipl.-Chem.
(86) International application number: PCT/JP2000/002348
(87) International publication number: WO 2000/061148

(56) References cited:
- EP-A- 0 968 716
- EP-A2- 0 289 746
- WO-A1-93/23082
- WO-A1-98/18471
- JP-A- 3 145 464
- MIYOKO OTA.: 'Endothelin - 1 katogan junkan shougai model no VEP ni taisuru atarashii Ca2 + kikkou-yaku tengan no kouka' JOURNAL OF OSAKA IKA DAIGAKU, vol. 57, no. 3, 1998, pages 50 - 57, XP002946619

## Description

### Technical Field

The present invention relates to an inhibitor of a visual function disturbance caused by an ophthalmic nerve cell disorder due to a factor other than an ophthalmic circulatory disorder, which inhibitor contains a specific 1,4-dihydropyridine derivative or an acid addition salt thereof. The present invention also relates to a method for inhibiting a visual function disturbance caused by optic nerve cell disorder due to a factor other than ophthalmic circulatory disorder. As used in this specification, the "optic nerve cell" includes, for example, retinal ganglion cell, rod, spindle, visual cell, bipolar cell, horizontal cell, amacrine cell, Müllerian cell, centrifugal nerve fiber, optic nerve cell and the like. The "optic" in the optic nerve cell refers to the retina and the tissue around the retina, and is a general designation of specifically the retina, optic nerve, blood vessel of retina, retinal pigment epithelium, optic disk, choroid, sclera and vitreous base. Of the above-mentioned tissues, particularly retina, blood vessel of retina, retinal pigment epithelium, optic disk and choroids are sometimes generally referred to as retinochoroids.

### Background Art

It is already known that a certain kind of Ca antagonist, 1,4-dihydropyridine derivative and an acid addition salt thereof, are useful as a hypotensive medicament, a therapeutic medicament of angina pectoris, a cerebral circulation improving medicament, a peripheral circulation improving agent, a renal function improving medicament, an anti-arterial sclerosis medicament, a smooth muscle relaxant, an antiallergic medicament, and a therapeutic medicament of cataract, a therapeutic medicament of glaucoma (JP-A-63-225355). In addition, this 1,4-dihydropyridine derivative is already known to be useful as an ophthalmic circulation disorder improving medicament (PCT/JP97/03866, WO98/18471).

Ophthalmic nerve cell disorders are caused by an ophthalmic circulatory disorder (intraocular blood circulation disorder) that causes an ophthalmic nerve cell disorder as described in the specification of the above-mentioned PCT application, as well as by many factors independent of an ophthalmic circulation disorder. For example, one of such major factors is an increase in the amount of calcium in optic nerve cells. A report has documented, with regard to the ophthalmic nerve cell disorders caused by the above-mentioned increase in the amount of calcium, that calcium antagonists, flunarizine and nifedipine, respectively inhibited ischemic retinal neurocyte disorder in a test using experiment models, such as high ocular pressure rat models and central retinal artery and vein ligation models [Takahashi K., Lam, T.T., Edward, D.P., Buchi, E.R., Tso, M., Arch. Ophthalmol. 110, 862-870 (1992). Crosson, C.E., Willis, J.A., Potter, D.E., J. Ocular Pharmacolol. 6. 293-299 (1990)].

Clinically, however, a visual function disturbance inhibitor based on an optic nerve cell-protective action of this calcium antagonist is not heretofore known, as the situation stands now. As far as the present inventors know, there is no report on successful inhibition of an ophthalmic nerve cell disorder in the above-mentioned experiment models, which is achieved by a calcium antagonist other than the above-mentioned flunarizine and nifedipine.

### Disclosure of the Invention

Under the circumstances, the present inventor has closely studied the efficacy of the Ca antagonist, 1,4-dihydropyridine derivative and an acid addition salt thereof to be mentioned later, and found that these compounds have a superior inhibitory action on a visual function disturbance caused by an ophthalmic nerve cell disorder due to a factor other than an ophthalmic circulatory disorder, and further studies have resulted in the completion of the present invention.

The present invention provides a novel use of a 1,4-dihydropyridine derivative and an acid addition salt thereof as an inhibitor of a visual function disturbance caused by an ophthalmic nerve cell disorder due to a factor other than an ophthalmic circulatory disorder. The present invention further provides a method for inhibiting a visual function disturbance caused by an ophthalmic nerve cell disorder due to a factor other than an ophthalmic circulatory disorder.

Accordingly, the present invention provides the following.
[1] An inhibitor of a visual function disturbance caused by an ophthalmic nerve cell disorder due to a factor other than an ophthalmic circulatory disorder, which contains, as an active ingredient, a 1,4-dihydropyridine derivative of the formula (I) 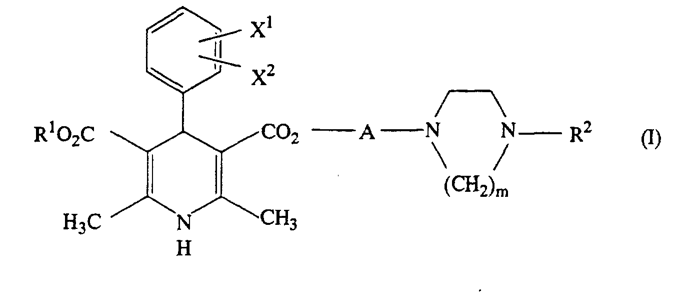 wherein X¹ and X² are the same or different and each is hydrogen atom, fluoromethyl, fluoromethoxy, halogen, cyano or nitro, R¹ is lower alkyl, R² is acyl, alkoxycarbonyl, acylalkyl, N-alkyl-substituted carbamoylalkyl, alkoxyalkyl, alkoxycarbonylalkyl, acyloxyalkyl, nitratoalkyl, cyanoalkyl, hetero ring-alkyl, haloalkyl, alkenyl or alkynyl, A is alkylene having 5 or more carbon atoms in total including a carbon atom bonded to two alkyls, and m is an integer of 1 to 3, or an acid addition salt thereof (hereinafter sometimes to be referred as an inventive compound).
[2] The visual function disturbance inhibitor of [1] above, wherein the ophthalmic nerve cell disorder is caused by an increased amount of calcium in a cell.
[3] The visual function disturbance inhibitor of [1] above, wherein R² is acylalkyl, N-alkyl-substituted carbamoylalkyl, alkoxyalkyl, cyanoalkyl, hetero ring-alkyl, haloalkyl, alkenyl or alkynyl.
[4] The visual function disturbance inhibitor of [1] above, wherein R² is alkenyl or alkynyl.
[5] The visual function disturbance inhibitor of [1] above, wherein A is alkylene having 5 to 10 carbon atoms in total including a carbon atom bonded to two alkyls.
[6] The visual function disturbance inhibitor of [1] above, wherein the 1,4-dihydropyridine derivative or an acid addition salt thereof is 3-(4-allyl-1-piperazinyl)-2,2-dimethylpropyl methyl 2,6-dimethyl-4-(m-nitrophenyl)-1,4-dihydropyridine-3,5-dicarboxylate dihydrochloride.
[7] The visual function disturbance inhibitor of any of [1] to [6] above, which is used for the prophylaxis or treatment of a retinochoroidal disease.
[8] The visual function disturbance inhibitor of [7] above, which is for topical administration to the eye.
[9] The visual function disturbance inhibitor of [8] above, which is an eye drop.
[10] The visual function disturbance inhibitor of [8] above, which is an eye ointment.
[11] A method for inhibiting a visual function disturbance caused by an ophthalmic nerve cell disorder due to a factor other than an ophthalmic circulatory disorder, which method comprises administering an effective amount of a 1,4-dihydropyridine derivative of the formula (I) 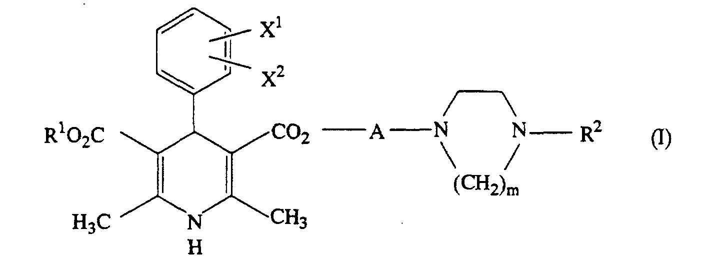 wherein X¹ and X² are the same or different and each is hydrogen atom, fluoromethyl, fluoromethoxy, halogen, cyano or nitro, R¹ is lower alkyl, R² is acyl, alkoxycarbonyl, acylalkyl, N-alkyl-substituted carbamoylalkyl, alkoxyalkyl, alkoxycarbonylalkyl, acyloxyalkyl, nitratoalkyl, cyanoalkyl, hetero ring-alkyl, haloalkyl, alkenyl or alkynyl, A is alkylene having 5 or more carbon atoms in total including a carbon atom bonded to two alkyls, and m is an integer of 1 to 3, or an acid addition salt thereof.
[12] The method of [11] above, wherein the ophthalmic nerve cell disorder is caused by an increased amount of calcium in a cell.
[13] The method of [11] above, wherein R² is acylalkyl, N-alkyl-substituted carbamoylalkyl, alkoxyalkyl, cyanoalkyl, hetero ring-alkyl, haloalkyl, alkenyl or alkynyl.
[14] The method of [11] above, wherein R² is alkenyl or alkynyl.
[15] The method of [11] above, wherein A is alkylene having 5 to 10 carbon atoms in total including a carbon atom bonded to two alkyls.
[16] The method of [11] above, wherein the 1,4-dihydropyridine derivative or an acid addition salt thereof is 3-(4-allyl-1-piperazinyl)-2,2-dimethylpropyl methyl 2,6-dimethyl-4-(m-nitrophenyl)-1,4-dihydropyridine-3,5-dicarboxylate dihydrochloride.
[17] The method of any of [11] to [16] above, which is used for the prophylaxis or treatment of retinochoroidal diseases.
[18] The method of [17] above, which comprises administering the 1,4-dihydropyridine derivative or an acid addition salt thereof topically to the eye.
[19] The method of [18] above, which comprises administering the 1,4-dihydropyridine derivative or an acid addition salt thereof in the form of an eye drop.
[20] The method of [18] above, which comprises administering the 1,4-dihydropyridine derivative or an acid addition salt thereof in the form of an eye ointment.
[21] Use of a 1,4-dihydropyridine derivative of the formula (I) 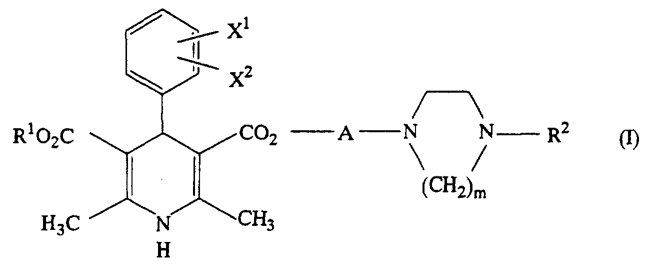 wherein X¹ and X² are the same or different and each is hydrogen atom, fluoromethyl, fluoromethoxy, halogen, cyano or nitro, R¹ is lower alkyl, R² is acyl, alkoxycarbonyl, acylalkyl, N-alkyl-substituted carbamoylalkyl, alkoxyalkyl, alkoxycarbonylalkyl, acyloxyalkyl, nitratoalkyl, cyanoalkyl, hetero ring-alkyl, haloalkyl, alkenyl or alkynyl, A is alkylene having 5 or more carbon atoms in total including a carbon atom bonded to two alkyls, and m is an integer of 1 to 3, or an acid addition salt thereof, for the production of a pharmaceutical agent for inhibiting visual function disturbance caused by an ophthalmic nerve cell disorder due to a factor other than an ophthalmic circulatory disorder.
[22] The use of [21] above, wherein the ophthalmic nerve cell disorder is caused by an increased amount of calcium in a cell.
[23] The use of [21] above, wherein R² is acylalkyl, N-alkyl-substituted carbamoylalkyl, alkoxyalkyl, cyanoalkyl, hetero ring-alkyl, haloalkyl, alkenyl or alkynyl.
[24] The use of [21] above, wherein R² is alkenyl or alkynyl.
[25] The use of [21] above, wherein A is alkylene having 5 to 10 carbon atoms in total including a carbon atom bonded to two alkyls.
[26] The use of [21] above, wherein the 1,4-dihydropyridine derivative or an acid addition salt thereof is 3-(4-allyl-1-piperazinyl)-2,2-dimethylpropyl methyl 2,6-dimethyl-4-(m-nitrophenyl)-1,4-dihydropyridine-3,5-dicarboxylate dihydrochloride.
[27] The use of any of [21] to [26] above, which is for the prophylaxis or treatment of a retinochoroidal disease.
[28] The use of [27] above, wherein the pharmaceutical agent for inhibiting visual function disturbance caused by an ophthalmic nerve cell disorder due to a factor other than an ophthalmic circulatory disorder is for topical administration to the eye.
[29] The use of [28] above, wherein the pharmaceutical agent for inhibiting visual function disturbance caused by an ophthalmic nerve cell disorder due to a factor other than an ophthalmic circulatory disorder is an eye drop.
[30] The use of [28] above, wherein the pharmaceutical agent for inhibiting visual function disturbance caused by an ophthalmic nerve cell disorder due to a factor other than an ophthalmic circulatory disorder is an eye ointment.
[31] A pharmaceutical composition for inhibiting a visual function disturbance caused by an ophthalmic nerve cell disorder due to a factor other than an ophthalmic circulatory disorder, which contains a 1,4-dihydropyridine derivative of the formula (I) 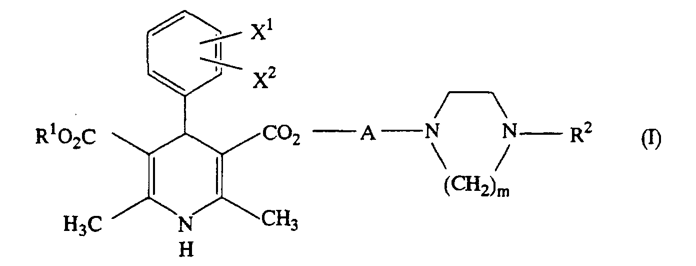 wherein X¹ and X² are the same or different and each is hydrogen atom, fluoromethyl, fluoromethoxy, halogen, cyano or nitro, R¹ is lower alkyl, R² is acyl, alkoxycarbonyl, acylalkyl, N-alkyl-substituted carbamoylalkyl, alkoxyalkyl, alkoxycarbonylalkyl, acyloxyalkyl, nitratoalkyl, cyanoalkyl, hetero ring-alkyl, haloalkyl, alkenyl or alkynyl, A is alkylene having 5 or more carbon atoms in total including a carbon atom bonded to two alkyls, and m is an integer of 1 to 3, or an acid addition salt thereof, and a pharmaceutically acceptable carrier.
[32] The pharmaceutical composition of [31] above, wherein the ophthalmic nerve cell disorder is caused by an increased amount of calcium in a cell.
[33] The pharmaceutical composition of [31] above, wherein R² is acylalkyl, N-alkyl-substituted carbamoylalkyl, alkoxyalkyl, cyanoalkyl, hetero ring-alkyl, haloalkyl, alkenyl or alkynyl.
[34] The pharmaceutical composition of [31] above, wherein R² is alkenyl or alkynyl.
[35] The pharmaceutical composition of [31] above, wherein A is alkylene having 5 to 10 carbon atoms in total including a carbon atom bonded to two alkyls.
[36] The pharmaceutical composition of [31] above, wherein the 1,4-dihydropyridine derivative or an acid addition salt thereof is 3-(4-allyl-1-piperazinyl)-2,2-dimethylpropyl methyl 2,6-dimethyl-4-(m-nitrophenyl)-1,4-dihydropyridine-3,5-dicarboxylate dihydrochloride.
[37] The pharmaceutical composition of any of [31] to [36] above, which is used for the prophylaxis or treatment of a retinochoroidal disease.
[38] The pharmaceutical composition of [37] above, which is for topical administration to the eye.
[39] The pharmaceutical composition of [38] above, which is in the form of an eye drop.
[40] The pharmaceutical composition of [38] above, which is in the form of an eye ointment.
[41] A commercial package comprising the pharmaceutical composition of [31] above and a written matter associated therewith, the written matter stating that the pharmaceutical composition can or should be used for inhibiting a visual function disturbance caused by an ophthalmic nerve cell disorder due to a factor other than an ophthalmic circulatory disorder.

### Brief Description of the Drawings

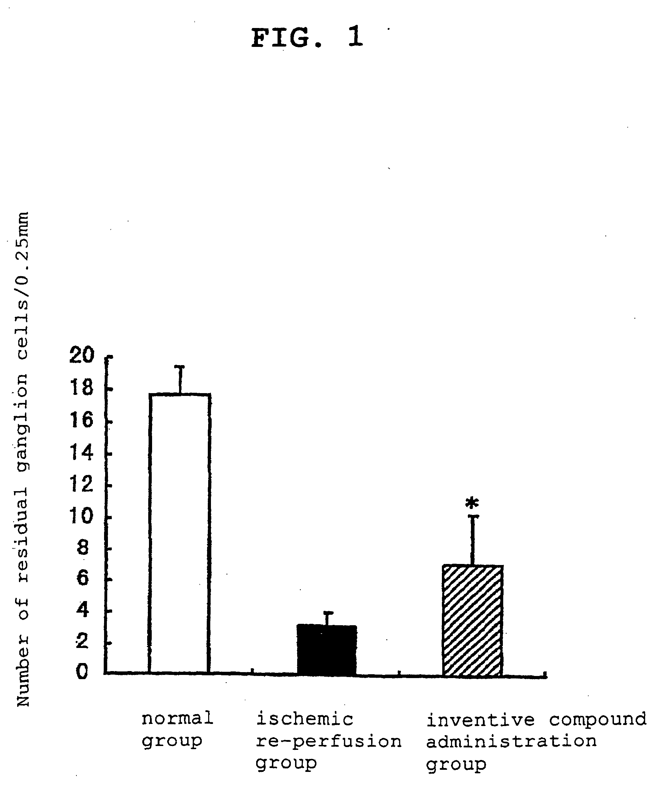

Fig. 1 is a graph showing an inhibitory effect of the inventive compound on the ophthalmic nerve cell disorder in rats, wherein the axis of ordinates shows the number of residual retinal ganglion cells in 0.25 mm of vertical retinal cross section at day 7 from the release from ischemia, at a certain distance from the optic disc and each value shows mean±standard error (n=6) with a significant difference from an ischemic re-perfusion group found at *P<0.027.

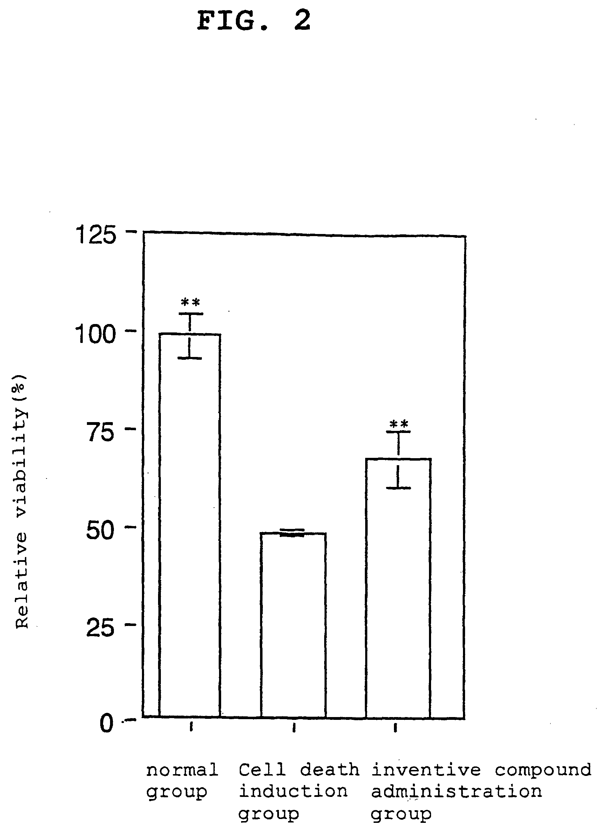

Fig. 2 is a graph showing an inhibitory effect of the inventive compound on an ophthalmic nerve cell disorder caused by an increased amount of calcium in the cell, wherein the axis of ordinates shows the viability (%) relative to that of a normal group, and wherein a significant difference from a cell death induction group is found at **P<0.01.

### Detailed Description of the Invention

The visual function disturbance inhibitor of the present invention is suitable as an inhibitor of a visual function disturbance caused by an ophthalmic nerve cell disorder due to a factor other than an ophthalmic circulatory disorder, particularly, an ophthalmic nerve cell disorder caused by an increased amount of calcium in the cell.

As mentioned earlier, an ophthalmic nerve cell disorder is caused by an ophthalmic circulatory disorder, as well as many factors independent of ophthalmic circulatory disorders. Examples of the above-mentioned factors include an increase in the amount of calcium in a cell. Therefore, an effectiveness on an ophthalmic nerve cell disorder caused by an ophthalmic circulatory disorder does not necessarily mean a simultaneous effectiveness in inhibiting a visual function disturbance caused by an ophthalmic nerve cell disorder due to a factor other than an ophthalmic circulatory disorder. Under the circumstances, the present inventors have newly found using a central retinal artery and vein ligation model, which is one of the experimental models of an increase in a calcium amount in an optic nerve cell, that the inventive compound effectively inhibits an ophthalmic nerve cell disorder due to a factor other than an ophthalmic circulatory disorder.

In the present specification, Cₓ means that the number of carbon atoms is X. For example, C₁₋₄ means that the number of carbon atoms is 1 to 4.

In the formula (I), fluoromethyl at X¹ and X² is monofluoromethyl, difluoromethyl or trifluoromethyl, fluoromethoxy is monofluoromethoxy, difluoromethoxy or trifluoromethoxy, and halogen is fluorine atom, chlorine atom, bromine atom or iodine atom.

In the formula (I), the lower alkyl at R¹ preferably has 1 to 4 carbon atoms, and may be any of linear, branched and cyclic. Examples thereof include methyl, ethyl, n-propyl, isopropyl, n-butyl, isobutyl, sec-butyl, tert-butyl, cyclopropylmethyl and the like.

In the formula (I), acyl at R² may be any of aliphatic acyl, aromatic acyl, heterocyclic acyl. The aliphatic acyl preferably has 1 to 5 carbon atoms, and may be linear or branched, and saturated or unsaturated. When it is unsaturated, it preferably has 1 or 2 double bond(s) or triple bond(s). The aromatic acyl or heterocyclic acyl may be that wherein a carbonyl group is directly bonded to an aromatic group or a heterocyclic group or that wherein a carbonyl group is bonded to an aromatic group via an aliphatic group (e.g., saturated or unsaturated and having 1 to 3 carbon atoms, and when it is unsaturated, it has 1 or 2 double bond(s) or triple bond(s)). The hetero ring is preferably a 5-or 6-membered ring, particularly that wherein the hetero atom is a nitrogen atom or an oxygen atom. The aliphatic group, aromatic group and heterocyclic group of aliphatic acyl, aromatic acyl and heterocyclic acyl may be substituted by halogen (chlorine atom, bromine atom and the like), hydroxyl group, carboxyl group, alkoxyl group, acyl group, acylamino group and the like.

Preferable examples of acyl group at R² include formyl, acetyl, crotonoyl, acryloyl, propioloyl, benzoyl, phenylacetyl, cinnamoyl, p-acetamino benzoyl, m-methoxybenzoyl, m-dimethylamino benzoyl, p-hydroxycinnamoyl, furoyl, nicotinoyl, piperidinomethylcarbonyl and the like.

The alkoxycarbonyl at R² is that having linear or branched C₁₋₅ alkoxy. Preferable examples include methoxycarbonyl, ethoxycarbonyl, propoxycarbonyl, tert-butoxycarbonyl and the like.

The acyl of acylalkyl at R² is exemplified by those mentioned above and alkyl is exemplified by linear or branched C₁₋₅ alkyl. Preferable examples include phenacyl, acetonyl, methylcarbonylethyl, pyrrolidinocarbonylmethyl and the like.

The N-alkyl-substituted carbamoylalkyl at R² may be any of mono-substituted and di-substituted. As the alkyl group of the substituent, linear or branched C₁₋₅ alkyl is exemplified. Preferable examples include methylcarbamoylmethyl, piperadinocarbamoylmethyl, dimethylcarbamoylmethyl and the like.

The alkoxy and alkyl of alkoxyalkyl at R² are exemplified by linear or branched C₁₋₅ alkoxy and C₁₋₅ alkyl. Preferable examples include methoxyethyl, ethoxyethyl, methoxypropyl and the like.

The alkoxy and alkyl of alkoxycarbonylalkyl at R² are exemplified by linear or branched C₁₋₅ alkoxy and C₁₋₅ alkyl. Preferable examples include methoxycarbonylmethyl, ethoxycarbonylmethyl, ethoxycarbonylethyl and the like.

The acyl of acyloxyalkyl at R² is exemplified by those mentioned above, and as the alkyl, linear or branched C₁₋₅ alkyl is exemplified. Preferable examples include acetoxyethyl, benzoyloxyethyl and the like.

The alkyl of nitratoalkyl at R² is exemplified by linear or branched C₁₋₅ alkyl. Preferable examples include nitratoethyl, nitratopropyl and the like.

The alkyl of cyanoalkyl at R² is exemplified by linear or branched C₁₋₅ alkyl. Preferable examples include cyanomethyl, cyanoethyl and the like.

The hetero ring of hetero ring-alkyl at R² is preferably exemplified by 5- or 6-membered ring, which particularly has a nitrogen atom or oxygen atom as the hetero atom. Examples thereof include piperidino, morpholino and the like. As the alkyl, linear or branched C₁₋₅ alkyl is exemplified. Preferable examples include piperidinoethyl, morpholinoethyl and the like.

The halogen of haloalkyl at R² is exemplified by fluorine atom, chlorine atom, bromine atom and the like, and as the alkyl, linear or branched C₁₋₅ alkyl is exemplified. Preferable examples include halogen-trisubstituted methyl (e.g., trifluoromethyl), halogen-monosubstituted ethyl (e.g., monofluoroethyl) and the like.

As the alkenyl and alkynyl at R² are exemplified by linear or branched C₂₋₅ alkenyl and C₂₋₅ alkynyl, such as vinyl, propenyl, isopropenyl, butenyl, ethynyl, propinyl, butynyl, pentinyl and the like.

In the formula (I), alkylene having 5 or more carbon atoms in total including a carbon atom bonded to two alkyls, which is represented by A, may be linear or branched, and preferably has 10 or less, particularly 8 or less, carbon atoms. Examples thereof include 2,2-dimethyltetramethylene, 2,2-dimethylpentamethylene, 2,2-dimethylhexamethylene, 2,2-dimethyltrimethylene, 1,1-dimethyltrimethylene and the like, with preference given to 2,2-dimethyltrimethylene.

In the formula (I), m is preferably 2 or 3.

The 1,4-dihydropyridine derivative of the formula (I) wherein R² is acylalkyl, N-alkyl-substituted carbamoylalkyl, alkoxyalkyl, cyanoalkyl, hetero ring-alkyl, haloalkyl, alkenyl or alkynyl (particularly, R² is alkenyl or alkynyl), and an acid addition salt thereof are particularly superior in pharmacological action and water solubility.

The acid addition salt of the inventive compound (I) includes, for example, inorganic salts such as hydrochloride, sulfate, hydrobromide and phosphate, and organic salts such as acetate, succinate, maleate, fumarate, malate, tartrate and methanesulfonate, and may be any as long as it is pharmacologically acceptable.

As the 1,4-dihydropyridine derivative of the formula (I) and an acid addition salt thereof to be contained in the inhibitor of a visual function disturbance caused by an ophthalmic nerve cell disorder due to a factor other than an ophthalmic circulatory disorder, of the present invention, the following compounds are exemplified.
(1) 3-(4-allyl-1-piperazinyl)-2,2-dimethylpropyl methyl 2,6-dimethyl-4-(m-nitrophenyl)-1,4-dihydropyridine-3,5-dicarboxylate dihydrochloride [general name: iganidipine hydrochloride]
(2) 3-[4-(1-propenyl)-1-piperazinyl]-2,2-dimethylpropyl methyl 2,6-dimethyl-4-(m-nitrophenyl)-1,4-dihydropyridine-3,5-dicarboxylate dihydrochloride
(3) 3-(4-cyanomethyl-1-piperazinyl)-2,2-dimethylpropyl methyl 2,6-dimethyl-4-(m-nitrophenyl)-1,4-dihydropyridine-3,5-dicarboxylate dihydrochloride
(4) 3-[4-(2-methyl-2-propenyl)-1-homopiperazinyl]-2,2-dimethylpropyl methyl 2,6-dimethyl-4-(m-nitrophenyl)-1,4-dihydropyridine-3,5-dicarboxylate dihydrochloride
(5) 3-(4-allyl-1-homopiperazinyl)-2,2-dimethylpropyl methyl 2,6-dimethyl-4-(m-nitrophenyl)-1,4-dihydropyridine-3,5-dicarboxylate dihydrochloride
(6) 3-[4-(tert-butyloxycarbonyl)-1-homopiperazinyl]-2,2-dimethylpropyl methyl 2,6-dimethyl-4-(m-nitrophenyl)-1,4-dihydropyridine-3,5-dicarboxylate
(7) 3-(4-formyl-1-homopiperazinyl)-2,2-dimethylpropyl methyl 2,6-dimethyl-4-(m-nitrophenyl)-1,4-dihydropyridine-3,5-dicarboxylate

The 1,4-dihydropyridine derivative or an acid addition salt thereof to be contained in the inhibitor of a visual function disturbance caused by an ophthalmic nerve cell disorder due to a factor other than an ophthalmic circulatory disorder, of the present invention can be synthesized as appropriate according to the method described in the above-mentioned JP-A-63-225355 or a method analogous thereto.

Examples of the object diseases subject to the prophylaxis or treatment with an inhibitor of a visual function disturbance caused by an ophthalmic nerve cell disorder due to a factor other than an ophthalmic circulatory disorder of the present invention include retinochoroidal diseases (e.g., abnormal blood vessel of retina (e.g., retinal vascular occlusion, retinal periphlebitis, Eales' disease, ischemic eye syndrome, retinal macroaneurysm etc.), retinopathy (e.g., hypertensive retinopathy, renal retinopathy, diabetic retinopathy etc.), pigmentary retinal epitheliosis, retinal dystrophy, macular dystrophy, retinochoroidal atrophy, chorioretinopathy, macular degeneration, macular edema, pigmentary retinal epithelial detachment, retinal detachment, degenerative retinoschisis, tumor (e.g., retinoblastoma, pigmentary retinal epithelial tumor, optic disc telangioma etc.), optic neurosis (e.g., ischemic optic neurosis etc.), optic disc swelling (e.g., papillary stasis, papilledema etc.) and the like), glaucoma with eye ground tissue cell disorder (e.g., open-angle glaucoma, low tension glaucoma, angle-closure glaucoma etc.), and complications in the posterior segment of the eye due to photocoagulation (macular edema, retinal detachment, optic neuritis, abnormal visual field, abnormal light sense, dyschromatosis etc.). As used herein, by the "complications in the posterior segment of the eye due to photocoagulation" is meant changes in the circulation in the part subjected to laser irradiation and the peripheral part thereof, which occurs during photocoagulation by laser irradiation, eye ground tissue cell disorders, such as inflammatory response due to the heat of coagulation and diseases induced by such disorders.

When the inventive compound is used as an inhibitor of a visual function disturbance caused by an ophthalmic nerve cell disorder due to a factor other than an ophthalmic circulatory disorder, one or more kinds of the inventive compounds can be contained on combination as appropriate, according to the object and need.

The 1,4-dihydropyridine derivative (I) and an acid addition salt thereof can be administered orally or parenterally as an inhibitor of a visual function disturbance caused by an ophthalmic nerve cell disorder due to a factor other than an ophthalmic circulatory disorder. The dosage form of the preparation includes, for example, solid preparation such as tablet, granule, powder, capsule, ointment and the like, and liquid preparation such as injection and eye drop, which can be prepared by a known method. Where necessary, these preparations may contain a pharmaceutically acceptable carrier and various additives for general use, such as excipient, binder, thickener, dispersing agent, reabsorption enhancer, buffer, surfactant, solubilizer, preservative, emulsifier, isotonicity agent, stabilizer, pH adjusting agent and the like.

When this preparation is used for inhibiting an inhibitor of a visual function disturbance caused by an ophthalmic nerve cell disorder due to a factor other than an ophthalmic circulatory disorder, this preparation is preferably administered topically to the eye for the prevention of side effects. Examples of the additive to be used for the eye drop include the following.

As the buffer, for example, phosphate, borate, citrate, tartrate, acetate, amino acid and the like are used (preferably buffer having buffer capacity in the range of pH 2-9). As the isotonicity agent, for example, saccharides such as sorbitol, glucose, mannitol and the like, polyhydric alcohols such as glycerin, polyethylene glycol, propylene glycol and the like, salts such as sodium chloride and the like can be used. As the preservative, for example, benzalkonium chloride, benzethonium chloride, paraoxybenzoates such as methyl paraoxybenzoate, ethyl paraoxybenzoate and the like, benzyl alcohol, phenethyl alcohol, sorbic acid and salts thereof, thimerosal, chlorobutanol and the like can be used. As the thickener, for example, hydroxyethylcellulose, hydroxypropylcellulose, methylcellulose, hydroxypropylmethylcellulose, carboxymethylcellulose, salts thereof and the like can be used. As the solubilizer (stabilizer), used are water-soluble polymers such as cyclodextrins, polyvinylpyrrolidone and the like, surfactants such as polysorbate 80 and the like. As the chelating agent, used are sodium edetate, sodium citrate, condensed sodium phosphate and the like. As the suspending agent, used are surfactants such as polysorbate 80 and the like, and water soluble polymers such as sodium methylcellulose, hydroxypropylmethylcellulose, methylcellulose and the like.

When the inventive compound is used as an inhibitor of a visual function disturbance caused by an ophthalmic nerve cell disorder due to a factor other than an ophthalmic circulatory disorder, for example, the dose is preferably 1 mg - 100 mg a day for an adult in the case of an injection, and 10 mg - 1000 mg (single dose) for an adult in the case of oral administration given several times a day, though subject to change depending on the kind of the compound to be used, the kind of the objective disease, the age and body weight of patients, applicable symptoms, dosage form and the like. When it is used as an eye drop, an eye drop having a concentration of 0.001 (w/v)% - 5 (w/v)% is preferably instilled several times a day by several drops per instillation for an adult, and when it is used as an eye ointment, an eye ointment having a concentration of 0.001 (w/v)% - 5 (w/v)% is preferably administered several times a day to an adult.

The inhibitor of a visual function disturbance caused by an ophthalmic nerve cell disorder due to a factor other than an ophthalmic circulatory disorder of the present invention may contain, where necessary, an inhibitor of a visual function disturbance caused by a different ophthalmic nerve cell disorder or other kinds of efficacious ingredients, as long as the object of the present invention is not impaired.

### Examples

The present invention is explained in more detail in the following by referring to Examples and Formulation Examples. The scope of the present invention is not limited by these examples.

### Example 1 Effect of the inventive compound on ophthalmic nerve cell disorder in rats

Effect of the inventive compound on an ophthalmic nerve cell disorder in rats was tested using a central retinal artery and vein ligation model, which is a model for examining increase in a calcium amount in an optic nerve cell.

### Test material

### Inventive compound: 3-(4-allyl-1-piperazinyl)-2,2-dimethylpropyl methyl 2,6-dimethyl-4-(m-nitrophenyl)-1,4-dihydropyridine-3,5-dicarboxylate dihydrochloride (general name iganidipine chloride)

### Test method

Male SD rats (body weight 150 g) were grouped into a normal group, ischemic re-perfusion group and inventive compound administration group. The ischemic re-perfusion group and the inventive compound administration group were anesthetized with pentobarbital 50 mg/kg, and the central retinal artery and vein were ligated with an arterial aneurysm clip for hemostasis for 55 minutes. At 7 days from declamping, eyeballs were removed and fixed with 4% formaldehyde to prepare a tissue sample. After embedding in paraffin according to a conventional method, a thin slice (2 µm thick) was prepared by horizontal section vertical to a surface of the retina including the optic disc, and stained with hematoxylin-eosin. The number of retinal ganglion cells per a 0.25 mm retina vertical section at a certain distance (1-2 mm) from the optic disc was counted under an optical microscope. As regards the normal group, the number of retinal ganglion cells was counted in the same manner only with exposure of the central retinal artery and vein and without hemostasis. Each thin slice was dissolved in physiological saline (5 µg/ml), and administered via the tail vein to the inventive compound administration group twice at 10 µg/kg of the inventive compound 15 min before hemostasis and immediately after declamping. Physiological saline was administered similarly to the normal group and ischemic re-perfusion group.

### Test results

The results are shown in Fig. 1. Significance was detected by the Student's t-Test.

As is clear from Fig. 1, the ischemic re-perfusion group showed a significant decrease in the retinal ganglion cell count after re-opening of blood flow as compared to the normal group, due to the ligation of the central retinal artery and vein once for hemostasis. This means that the hemostasis increased the amount of calcium in the retinal ganglion cells and, when the blood flow was re-opened, the retinal ganglion cell count decreased due to an ophthalmic nerve cell disorder caused by the above-mentioned increase in the amount of calcium. In contrast, the inventive compound administration group significantly inhibited decrease in the retinal ganglion cell count as compared to the ischemic re-perfusion group. The results clarified that the inventive compound improved an ophthalmic nerve cell disorder caused by an increased intracellular calcium amount.

### Example 2 Effect of the inventive compound on cell death induction by increased intracellular calcium amount

### Test material

### Inventive compound: 3-(4-allyl-1-piperazinyl)-2,2-dimethylpropyl methyl 2,6-dimethyl-4-(m-nitrophenyl)-1,4-dihydropyridine-3,5-dicarboxylate dihydrochloride (general name iganidipine chloride)

### Test methods

The retina was obtained from ten 8-day-old rats and treated with papain to give a cell suspension. Using a non-coat dish (TERUMO) and a dish (CORNING) coated with Thy-1.1 antibody (American Type Culture Collection, T11D7E2), retinal ganglion cells alone were isolated from the whole cells of the retina. The retinal ganglion cells on the dish were trypsinized and recovered.

Then the recovered retinal ganglion cells were plated on a 96 well plate coated with poly-D-lysine and laminin at a concentration of 5000 cells/well/100 µl. The isolated retinal ganglion cells were divided for the normal group, cell death induction group and inventive compound administration group. As the cultures of the cell death induction group and inventive compound administration group, calcium chloride was added to Neurobasal medium (GIBCO) at a final concentration of 5 mM to adjust its pH to 6.0 and used. As the additives to the culture, 40 ng/ml BDNF (Pepro Tech), 40 ng/ml CNTF (Sigma), 5 µM Forskolin (Sigma), 1 mM glutamine (Wako), 50x B27 supplement (GIBCO) and Pen-strep (GIBCO) were added.

For the inventive compound administration group, the inventive compound was added simultaneously with the plating of the cells and pre-incubated at 37°C for 1 h. After 1 h, kainic acid having a final concentration of 200 µM was added and the mixture was incubated at 37°C to induce cell death. The cell death induction group was treated in the same manner as was the inventive compound administration group except that the inventive compound was not added to induce cell death. In the normal group, kainic acid was not added. The cell death was judged by adding an MTT reagent, incubating for 1 h, and counting cells stained in dark blue as viable cells.

### Test results

The results are shown in Fig. 2. As is clear from Fig. 2, the cell death induction group showed a significant decrease in the retinal ganglion cell count as compared to the normal group, due to the cell death induction by increasing the amount of calcium in the cell by adding kainic acid. In contrast, the inventive compound administration group significantly inhibited decrease in the retinal ganglion cell count by the cell death induction due to the increased amount of calcium in the cell. The results clarified that the inventive compound improved an ophthalmic nerve cell disorder due to an increased calcium amount.

### Formulation Example 1

| | |
|---|---|
| iganidipine hydrochloride | 0.1 g |
| sodium acetate | 0.1 g |
| sodium chloride | 0.9 g |
| benzalkonium chloride | 0.005 g |
| acetic acid | appropriate amount |
| distilled water | total amount 100 ml |
| | pH 5.0 |

The above-mentioned ingredients are mixed by a conventional method to give an eye drop.

### Formulation Example 2

| | |
|---|---|
| iganidipine hydrochloride | 0.05 g |
| sodium acetate | 0.1 g |
| sodium chloride | 0.9 g |
| benzalkonium chloride | 0.005 g |
| acetic acid | appropriate amount |
| distilled water | total amount 100 ml |
| | pH 5.0 |

The above-mentioned ingredients are mixed by a conventional method to give an eye drop.

### Formulation Example 3

| | |
|---|---|
| iganidipine hydrochloride | 0.1 g |
| liquid paraffin | 10 g |
| sterile purified water appropriate amount | total amount 100 g |

The above-mentioned ingredients are mixed by a conventional method to give an eye ointment.

### Industrial Applicability

The 1,4-dihydropyridine derivative and an acid addition salt thereof contained in the preparation of the present invention are superior in water solubility and exhibit a superior inhibitory effect on a visual function disturbance caused by an ophthalmic nerve cell disorder due to a factor other than an ophthalmic circulatory disorder. Particularly, by topical administration of the inventive compound to the eye, a superior inhibitory effect on a visual function disturbance caused by an ophthalmic nerve cell disorder due to a factor other than an ophthalmic circulatory disorder can be beneficially afforded.

## Claims

1. Use of a 1,4-dihydropyridine derivative of the formula (I) 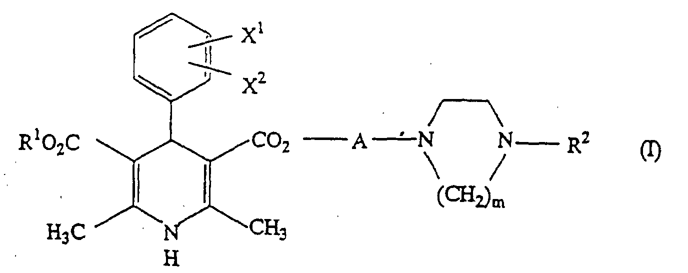 wherein X¹ and X² are the same or different and each is hydrogen atom, fluoromethyl, fluoromethoxy, halogen, cyano or nitro, R¹ is alkyl, R² is acyl, alkoxycarbonyl, acylalkyl, N-alkyl-substituted carbamoylalkyl, alkoxyalkyl, alkoxycarbonylalkyl, acyloxyalkyl, nitratoalkyl, cyanoalkyl, hetero ring-alkyl, haloalkyl, alkenyl or alkynyl, A is alkylene having 5 or more carbon atoms in total including a carbon atom bonded to two alkyls, and m is an integer of 1 to 3, or an acid addition salt thereof, for the production of a pharmaceutical agent for inhibiting visual function disturbance caused by an ophthalmic nerve cell disorder due to a factor other than an ophthalmic circulatory disorder wherein said visual function disturbance is caused by a disease selected from the group consisting of retinal periphlebitis, Eales' disease, ischemic eye syndrome, retinal macroaneurysm, hypertensive retinopathy, renal retinopathy, pigmentary retinal epitheliosis, retinal dystrophy, macular dystrophy, retinochoroidal atrophy, chorioretinopathy, macular edema, pigmentary retinal epithelial detachment, degenerative retinoschisis, retinoblastoma, pigmentary retinal epithelial tumor and optic disc telangioma, optic disc swelling and macular edema, retinal detachment, abnormal visual field, abnormal light sense and dyschromatosis due to photocoagulation.

2. The use of claim 1, wherein the ophthalmic nerve cell disorder is caused by an increased amount of calcium in a cell.

3. The use of claim 1, wherein R² is acylalkyl, N-alkyl-substituted carbamoylalkyl, alkoxyalkyl, cyanoalkyl, hetero ring-alkyl, haloalkyl, alkenyl or alkynyl.

4. The use of claim 1, wherein R² is alkenyl or alkynyl.

5. The use of claim 1, wherein A is alkylene having 5 to 10 carbon atoms in total including a carbon atom bonded to two alkyls.

6. The use of claim 1, wherein the 1,4-dihydropyridine derivative or an acid addition salt thereof is 3-(4-allyl-1-piperazinyl)-2,2-dimethylpropyl methyl 2,6-dimethyl-4-(m-nitrophenyl)-1,4-dihydropyridine-3,5-dicarboxylate dihydrochloride.

7. The use of any of claim 1 to claim 6, which is for the prophylaxis or treatment of a retinochoroidal disease.

8. The use of claim 7, wherein the pharmaceutical agent for inhibiting visual function disturbance caused by an ophthalmic nerve cell disorder due to a factor other than an ophthalmic circulatory disorder is for topical administration to the eye.

9. The use of claim 8, wherein the pharmaceutical agent for inhibiting visual function disturbance caused by an ophthalmic nerve cell disorder due to a factor other than an ophthalmic circulatory disorder is an eye drop.

10. The use of claim 8, wherein the pharmaceutical agent for inhibiting visual function disturbance caused by an ophthalmic nerve cell disorder due to a factor other than an ophthalmic circulatory disorder is an eye ointment.

11. Use of a 1,4-dihydropyridine derivative of the formula (I) 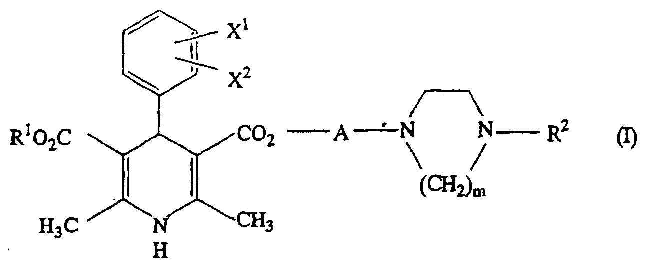 wherein X¹ and X² are the same or different and each is hydrogen atom, fluoromethyl, fluoromethoxy, halogen, cyano or nitro, R¹ is lower alkyl, R² is acyl, alkoxycarbonyl, acylalkyl, N-alkyl-substituted carbamoylalkyl, alkoxyalkyl, alkoxycarbonylalkyl, acyloxyalkyl, nitratoalkyl, cyanoalkyl, hetero ring-alkyl, haloalkyl, alkenyl or alkynyl, A is alkylene having 5 or more carbon atoms in total including a carbon atom bonded to two alkyls, and m is an integer of 1 to 3, or an acid addition salt thereof, for the production of a pharmaceutical agent for inhibiting visual function disturbance caused by an ophthalmic nerve cell disorder due to a factor other than an ophthalmic circulatory disorder, wherein said visual function disturbance is caused by a disease selected from the group consisting of pigmentary retinal epithelial detachment, retinal detachment and degenerative retinoschisis.

12. Use of a 1,4-dihydropyridine derivative of the formula (I) 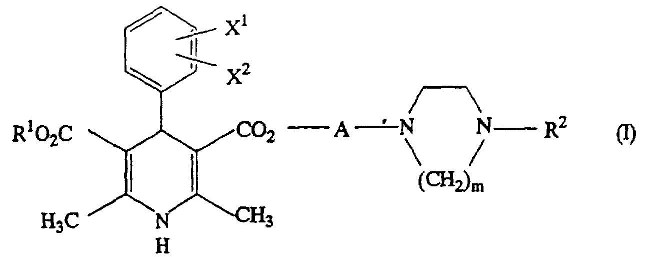 wherein X¹ and X² are the same or different and each is hydrogen atom, fluoromethyl, fluoromethoxy, halogen, cyano or nitro, R¹ is lower alkyl, R² is acyl, alkoxycarbonyl, acylalkyl, N-alkyl-substituted carbamoylalkyl, alkoxyalkyl, alkoxycarbonylalkyl, acyloxyalkyl, nitratoalkyl, cyanoalkyl, hetero ring-alkyl, haloalkyl, alkenyl or alkynyl, A is alkylene having 5 or more carbon atoms in total including a carbon atom bonded to two alkyls, and m is an integer of 1 to 3, or an acid addition salt thereof, for the production of a pharmaceutical agent for inhibiting visual function disturbance caused by an ophthalmic nerve cell disorder due to a factor other than an ophthalmic circulatory disorder, wherein said visual function disturbance is caused by a disease selected from the group consisting of retinoblastoma, pigmentary retinal epithelial tumor and optic disc telangioma.

13. Use of a 1,4-dihydropyridine derivative of the formula (I) 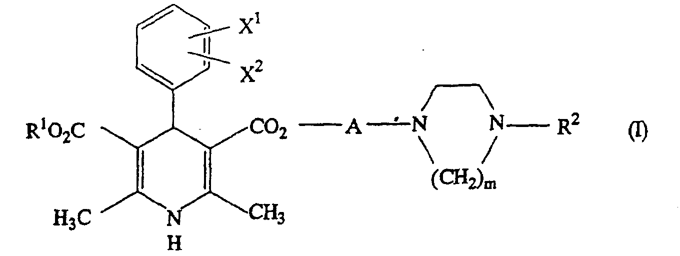 wherein X¹ and X² are the same or different and each is hydrogen atom, fluoromethyl, fluoromethoxy, halogen, cyano or nitro, R¹ is lower alkyl, R² is acyl, alkoxycarbonyl, acylalkyl, N-alkyl-substituted carbamoylalkyl, alkoxyalkyl, alkoxycarbonylalkyl, acyloxyalkyl, nitratoalkyl, cyanoalkyl, hetero ring-alkyl, haloalkyl, alkenyl or alkynyl, A is alkylene having 5 or more carbon atoms in total including a carbon atom bonded to two alkyls, and m is an integer of 1 to 3, or an acid addition salt thereof, for the production of a pharmaceutical agent for inhibiting visual function disturbance caused by an ophthalmic nerve cell disorder due to a factor other than an ophthalmic circulatory disorder. wherein said visual function disturbance is caused by a disease selected from the group consisting of macular edema, retinal detachment, abnormal visual field, abnormal light sense and dyschromatosis due to photocoagulation.

## Patentansprüche

1. Verwendung eines 1,4-Dihydropyridinderivats der Formel (I) 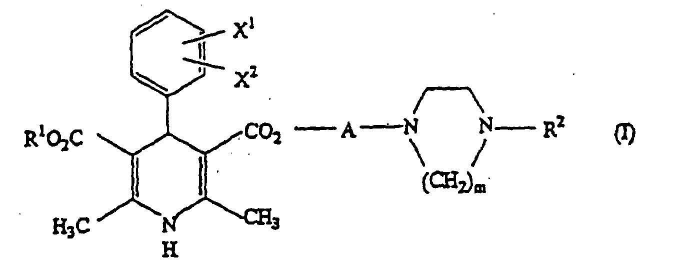 worin X¹ und X² gleich oder verschieden sind und jeweils ein Wasserstoffatom, Fluormethyl, Fluormethoxy, Halogen, Cyan oder Nitro sind, R¹ Alkyl ist, R² Acyl, Alkoxycarbonyl, Acylalkyl, N-alkylsubstituiertes Carbamoylalkyl, Alkoxyalkyl, Alkoxycarbonylalkyl, Acyloxyalkyl, Nitratoalkyl, Cyanalkyl, Heteroringalkyl, Halogenalkyl, Alkenyl oder Alkinyl ist, A Alkylen mit einschließlich eines an zwei Alkyl gebundenen Kohlenstoffatoms insgesamt 5 oder mehr Kohlenstoffatomen ist und m eine ganze Zahl von 1 bis 3 ist, oder eines Säureadditionssalzes davon zur Herstellung eines pharmazeutischen Mittels zum Hemmen der Sehfunktionsstörung, die durch eine Sehnervenzellenstörung hervorgerufen wird, die auf einen anderen Faktor als eine Augenkreislaufstörung zurückzuführen ist, wobei die Sehfunktionsstörung durch eine Krankheit hervorgerufen wird, die aus der aus Periphlebitis retinae, Eales-Syndrom, okulärem Ischämiesyndrom, retinalem Makroaneurysma, Retinopathia hypertonica, Retinopathia renalis, retinaler Pigmentepitheliose, Retinadystrophie, Makuladystrophie, Chorioretinitisatrophie, Chorioretinopathie, Makulaödem, Netzhautablösung bei Pigmentepitheliose, degenerative Retinoschisis, Retinoblastom, Tumor bei retinaler Pigmentepitheliose, Telangiom des Discus nervi optici, Schwellung des Discus nervi optici und Makulaödem, Netzhautablösung, abnormalem Sehfeld, abnormalem Lichtempfinden und Dyschromatose aufgrund von Lichtkoagulation bestehenden Gruppe ausgewählt ist.

2. Verwendung des Anspruchs 1, wobei die Sehnervenzellenstörung durch eine erhöhte Menge Calcium in einer Zelle verursacht wird.

3. Verwendung des Anspruchs 1, wobei R² Acylalkyl, N-alkylsubstituiertes Carbamoylalkyl, Alkoxyalkyl, Cyanalkyl, Heteroringalkyl, Halogenalkyl, Alkenyl oder Alkinyl ist.

4. Verwendung des Anspruchs 1, wobei R² Alkenyl oder Alkinyl ist.

5. Verwendung des Anspruchs 1, wobei A Alkylen mit einschließlich eines an zwei Alkyl gebundenen Kohlenstoffatoms insgesamt 5 bis 10 Kohlenstoffatomen ist.

6. Verwendung des Anspruchs 1, wobei das 1,4-Dihydropyridinderivat oder ein Säureadditionssalz davon 3-(4-Allyl-1-piperazinyl)-2,2-dimethylpropylmethyl-2,6-dimethyl-4-(m-nitrophenyl)-1,4-dihydropyridin-3,5-dicarboxylatdihydrochlorid ist.

7. Verwendung eines der Ansprüche 1 bis Anspruch 6 zur Prophylaxe oder Behandlung einer chorioretinalen Erkrankung.

8. Verwendung des Anspruchs 7, wobei das pharmazeutische Mittel zum Hemmen der Sehfunktionsstörung, die durch eine Sehnervenzellenstörung hervorgerufen wird, die auf einen anderen Faktor als eine Augenkreislaufstörung zurückzuführen ist, zur topischen Verabreichung an das Auge bestimmt ist.

9. Verwendung des Anspruchs 8, wobei das pharmazeutische Mittel zum Hemmen der Sehfunktionsstörung, die durch eine Sehnervenzellenstörung hervorgerufen wird, die auf einen anderen Faktor als eine Augenkreislaufstörung zurückzuführen ist, Augentropfen ist.

10. Verwendung des Anspruchs 8, wobei das pharmazeutische Mittel zum Hemmen der Sehfunktionsstörung, die durch eine Sehnervenzellenstörung hervorgerufen wird, die auf einen anderen Faktor als eine Augenkreislaufstörung zurückzuführen ist, eine Augensalbe ist.

11. Verwendung eines 1,4-Dihydropyridinderivats der Formel (I) 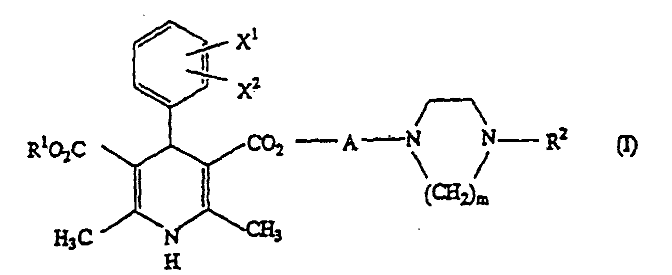 worin X¹ und X² gleich oder verschieden sind und jeweils ein Wasserstoffatom, Fluormethyl, Fluormethoxy, Halogen, Cyan oder Nitro sind, R¹ Alkyl ist, R² Acyl, Alkoxycarbonyl, Acylalkyl, N-alkylsubstituiertes Carbamoylalkyl, Alkoxyalkyl, Alkoxycarbonylalkyl, Acyloxyalkyl, Nitratoalkyl, Cyanalkyl, Heteroringalkyl, Halogenalkyl, Alkenyl oder Alkinyl ist, A Alkylen mit einschließlich eines an zwei Alkyl gebundenen Kohlenstoffatoms insgesamt 5 oder mehr Kohlenstoffatomen aufweist und m eine ganze Zahl von 1 bis 3 ist, oder eines Säureadditionssalzes davon zur Herstellung eines pharmazeutischen Mittels zum Hemmen der Sehfunktionsstörung, die durch eine Sehnervenzellenstörung hervorgerufen wird, die auf einen anderen Faktor als eine Augenkreislaufstörung zurückzuführen ist, wobei die Sehfunktionsstörung durch eine Krankheit verursacht wird, die aus der aus Netzhautablösung bei Pigmentepitheliose, Netzhautablösung und degenerativer Retinoschisis bestehenden Gruppe ausgewählt ist.

12. Verwendung eines 1,4-Dihydropyridinderivats der Formel (I) 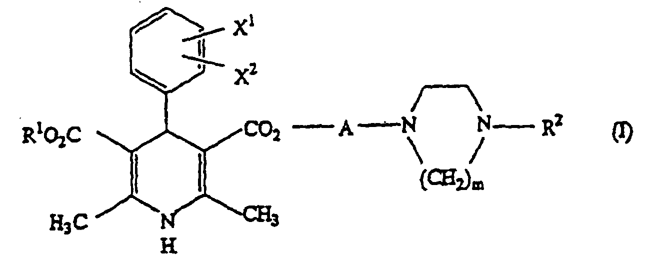 worin X¹ und X² gleich oder verschieden sind und jeweils ein Wasserstoffatom, Fluormethyl, Fluormethoxy, Halogen, Cyan oder Nitro sind, R¹ Alkyl ist, R² Acyl, Alkoxycarbonyl, Acylalkyl, N-alkylsubstituiertes Carbamoylalkyl, Alkoxyalkyl, Alkoxycarbonylalkyl, Acyloxyalkyl, Nitratoalkyl, Cyanalkyl, Heteroringalkyl, Halogenalkyl, Alkenyl oder Alkinyl ist, A Alkylen mit einschließlich eines an zwei Alkyl gebundenen Kohlenstoffatoms insgesamt 5 oder mehr Kohlenstoffatomen aufweist und m eine ganze Zahl von 1 bis 3 ist, oder eines Säureadditionssalzes davon zur Herstellung eines pharmazeutischen Mittels zum Hemmen der Sehfunktionsstörung, die durch eine Sehnervenzellenstörung hervorgerufen wird, die auf einen anderen Faktor als eine Augenkreislaufstörung zurückzuführen ist, wobei die Sehfunktionsstörung durch eine Krankheit verursacht wird, die aus der aus Retinoblastom, Tumor bei retinaler Pigmentepitheliose und Telangiom des Discus nervi optici bestehenden Gruppe ausgewählt ist.

13. Verwendung eines 1,4-Dihydropyridinderivats der Formel (I) 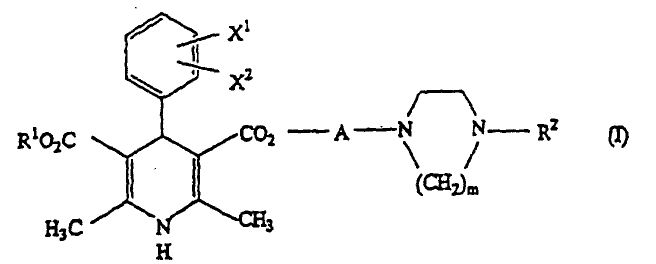 worin X¹ und X² gleich oder verschieden sind und jeweils ein Wasserstoffatom, Fluormethyl, Fluormethoxy, Halogen, Cyan oder Nitro sind, R¹ Alkyl ist, R² Acyl, Alkoxycarbonyl, Acylalkyl, N-alkylsubstituiertes Carbamoylalkyl, Alkoxyalkyl, Alkoxycarbonylalkyl, Acyloxyalkyl, Nitratoalkyl, Cyanalkyl, Heteroringalkyl, Halogenalkyl, Alkenyl oder Alkinyl ist, A Alkylen mit einschließlich eines an zwei Alkyl gebundenen Kohlenstoffatoms insgesamt 5 oder mehr Kohlenstoffatomen aufweist und m eine ganze Zahl von 1 bis 3 ist, oder eines Säureadditionssalzes davon zur Herstellung eines pharmazeutischen Mittels zum Hemmen der Sehfunktionsstörung, die durch eine Sehnervenzellenstörung hervorgerufen wird, die auf einen anderen Faktor als eine Augenkreislaufstörung zurückzuführen ist, wobei die Sehfunktionsstörung durch eine Krankheit verursacht wird, die aus der aus Makulaödem, Netzhautablösung, abnormalem Sehfeld, abnormalem Lichtempfinden und Dyschromatose aufgrund von Lichtkoagulation bestehenden Gruppe ausgewählt ist.

## Revendications

1. Utilisation d'un dérivé de la 1,4-dihydropyridine de formule (I) 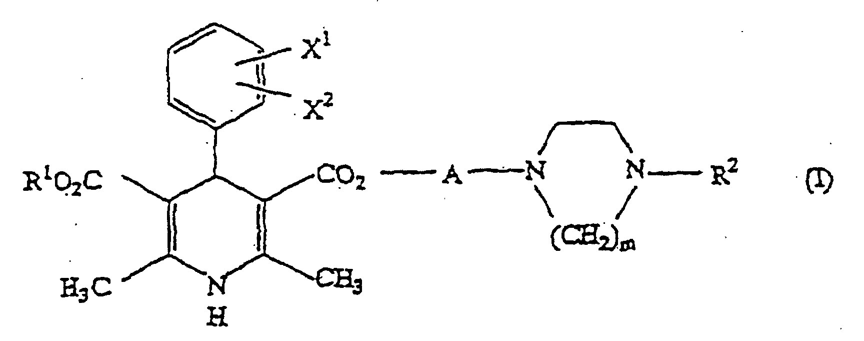 dans laquelle X¹ et X² sont identiques ou différents et chacun est un atome d'hydrogène, un fluorométhyle, un fluorométhoxy, un halogène, un cyano ou un nitro, R¹ est un alkyle, R² un acyle, un alcoxycarbonyle, un acylalkyle, un carbamoylalkyle N-alkyl-substitué un alcoxyalkyle, un alcoxycarbonylalkyle, un acyloxyalkyle, un nitratoalkyle, un cyanoalkyle, un hétéro alkyle porté par le noyau, un haloalkyle, un alcényle ou un alcynyle, A est un alkylène ayant 5 atomes de carbone ou plus au total comprenant un atome de carbone lié à deux alkyles, et m est un nombre entier de 1 à 3, ou un sel d'addition d'acide de celui-ci, pour la production d'un agent pharmaceutique pour l'inhibition de troubles de la fonction visuelle causés par un trouble des cellules nerveuses ophtalmiques dû à un facteur autre qu'un trouble circulatoire ophtalmique dans lequel ledit trouble de la fonction visuelle est causé par une affection choisie parmi le groupe constitué de la périphlébite rétinienne, de la maladie de Eales, de la rétinopathie ischémique, du macro-anévrisme rétinien, de la rétinopathie hypertensive, de la rétinopathie néphritique, de l'épithéliopathie rétinienne pigmentaire, de la dystrophie rétinienne, de la dystrophie maculaire, de l'atrophie rétino-choroïdienne, de la choriorétinopathie, de l'oedème maculaire, du décollement de l'épithélium rétinien pigmentaire, du rétinoschisis dégénératif, du rétinoblastome, de la tumeur de l'épithélium rétinien pigmentaire et de l'angiome plan du disque optique, de la tuméfaction du disque optique et de l'oedème maculaire, du décollement de la rétine, d'un champ visuel anormal, d'un sens lumineux anormal et d'une dyschromatose causée par photocoagulation.

2. Utilisation selon la revendication 1, dans laquelle le trouble des cellules nerveuses ophtalmiques est causé par une quantité accrue de calcium dans une cellule.

3. Utilisation selon la revendication 1, dans laquelle R² est un acylalkyle, un N-alkyl-substitué carbamoylalkyle, un alcoxyalkyle, un cyanoalkyle, un hétéro alkyle porté par le noyau, un haloalkyle, un alcényle ou un alcynyle.

4. Utilisation selon la revendication 1, dans laquelle R² est un alcényle ou un alcynyle.

5. Utilisation selon la revendication 1, dans laquelle A est un alkylène ayant de 5 à 10 atomes de carbone au total comprenant un atome de carbone lié à deux alkyles.

6. Utilisation selon la revendication 1, dans laquelle le dérivé de la 1,4-dihydropyridine ou un sel d'addition d'acide de celui-ci est 3-(4-allyl-1-pipérazinyl)-2,2-diméthylpropyl méthyl 2,6-diméthyl-9-(m-nitrophényl)-1,4-dihydropyridine-3,5-dicarboxylate dihydrochlorure.

7. Utilisation selon l'une quelconque des revendications 1 à 6, qui est pour la prophylaxie ou le traitement d'une affection rétinochoroïdienne.

8. Utilisation selon la revendication 7, dans laquelle l'agent pharmaceutique pour l'inhibition du trouble de la fonction visuelle causé par un trouble des cellules nerveuses ophtalmiques dû à un facteur autre qu'un trouble circulatoire ophtalmique est pour une administration topique sur l'oeil.

9. Utilisation selon la revendication 8, dans laquelle l'agent pharmaceutique pour l'inhibition du trouble de la fonction visuelle causé par un trouble des cellules nerveuses ophtalmiques dû à un facteur autre qu'un trouble circulatoire ophtalmique est un collyre.

10. Utilisation selon la revendication 8, dans laquelle l'agent pharmaceutique pour l'inhibition du trouble de la fonction visuelle causé par un trouble des cellules nerveuses ophtalmiques dû à un facteur autre qu'un trouble circulatoire ophtalmique est une pommade ophtalmique.

11. Utilisation d'un dérivé de la 1,4-dihydropyridine de formule (I) 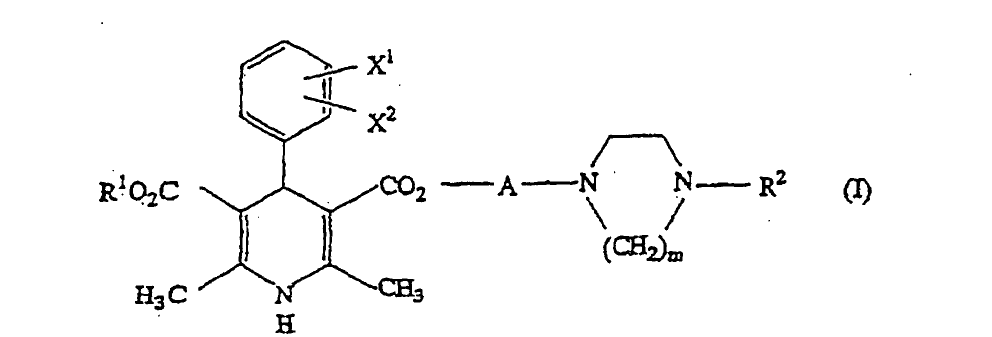 dans laquelle X¹ et X² sont identiques ou différents et chacun est un atome d'hydrogène, un fluorométhyle, un fluorométhoxy, un halogène, un cyano ou un nitro, R¹ est un alkyle inférieur, R² est un acyle, un alcoxycarbonyle, un acylalkyle, un N-alkyl-substitué carbamoylalkyle, un alcoxyalkyle, un alcoxycarbonylalkyle, un acyloxyalkyle, un nitratoalkyle, un cyanoalkyle, un hétéro alkyle porté par le noyau, un haloalkyle, un alcényle ou un alcynyle, A est un alkylène ayant 5 atomes de carbone ou plus au total comprenant un atome de carbone lié à deux alkyles, et m est un nombre entier de 1 à 3, ou un sel d'addition d'acide de celui-ci, pour la production d'un agent pharmaceutique pour l'inhibition d'un trouble de la fonction visuelle causé par un trouble des cellules nerveuses ophtalmiques dû à un facteur autre qu'un trouble circulatoire ophtalmique, dans lequel ledit trouble de la fonction visuelle est causé par une affection choisie parmi le groupe constitué du décollement de l'épithélium rétinien pigmentaire, du décollement de la rétine et du rétinoschisis dégénératif.

12. Utilisation d'un dérivé de la 1,4-dihydropyridine de formule (I) 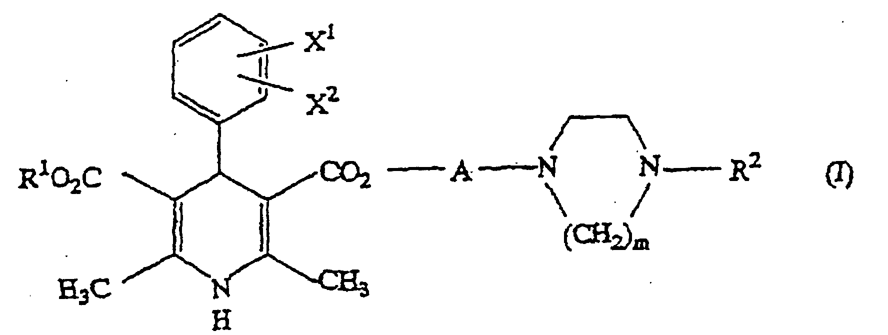 dans laquelle X¹ et X² sont identiques ou différents et chacun est un atome d'hydrogène, un fluorométhyle, un fluorométhoxy, un halogène, un cyano ou un nitro, R¹ est un alkyle inférieur, R² est un acyle, un alcoxycarbonyle, un acylalkyle, un N-alkyl-substitué carbamoylalkyle, un alcoxyalkyle, un alcoxycarbonylalkyle, un acyloxyalkyle, un nitratoalkyle, un cyanoalkyle, un hétéro alkyle porté par le noyau, un haloalkyle, un alcényle ou un alcynyle, A est un alkylène ayant 5 atomes de carbone ou plus au total comprenant un atome de carbone lié à deux alkyles, et m est un nombre entier de 1 à 3, ou un sel d'addition d'acide de celui-ci, pour la production d'un agent pharmaceutique pour l'inhibition d'un trouble de la fonction visuelle causé par un trouble des cellules nerveuses ophtalmiques dû à un facteur autre qu'un trouble circulatoire ophtalmique, dans lequel ledit trouble de la fonction visuelle est causé par une affection choisie parmi le groupe constitué du rétinoblastome, de la tumeur de l'épithélium rétinien pigmentaire et de l'angiome plan du disque optique.

13. Utilisation d'un dérivé de la 1,4-dihydropyridine de formule (I) 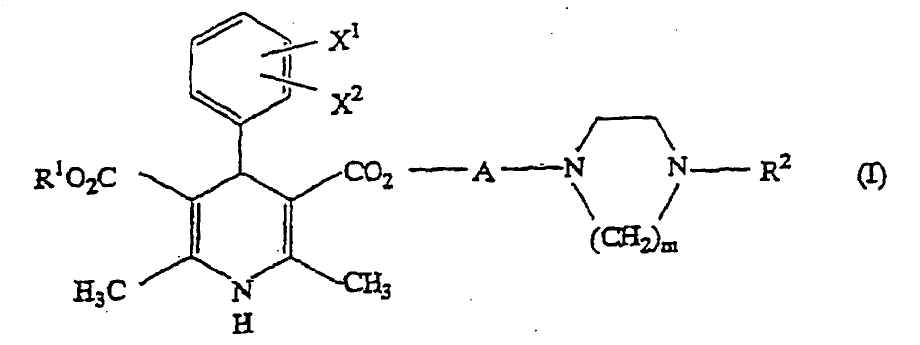 dans laquelle X¹ et X² sont identiques ou différents et chacun est un atome d'hydrogène, un fluorométhyle, un fluorométhoxy, un halogène, un cyano ou un nitro, R¹ est un alkyle inférieur, R² est un acyle, un alcoxycarbonyle, un acylalkyle, un N-alkyl-substitué carbamoylalkyle, un alcoxyalkyle, un alcoxycarbonylalkyle, un acyloxyalkyle, un nitratoalkyle, un cyanoalkyle, un hétéro alkyle porté par le noyau, un haloalkyle, un alcényle ou un alcynyle, A est un alkylène ayant 5 atomes de carbone ou plus au total comprenant un atome de carbone lié à deux alkyles, et m est un nombre entier de 1 à 3, ou un sel d'addition d'acide de celui-ci, pour la production d'un agent pharmaceutique pour l'inhibition d'un trouble de la fonction visuelle causé par un trouble des cellules nerveuses ophtalmiques dû à un facteur autre qu'un trouble circulatoire ophtalmique, dans lequel ledit trouble de la fonction visuelle est causé par une affection choisie parmi le groupe constitué de l'oedème maculaire, du décollement de la rétine, d'un champ visuel anormal, d'un sens lumineux anormal et d'une dyschromatose causée par photocoagulation.
